# EUROPEAN PATENT SPECIFICATION

(11) **EP 4 321 183 B1**
(45) Date of publication and mention of the grant of the patent: **18.03.2026**
(21) Application number: 22859512.0
(22) Date of filing: 10.06.2022
(51) Int. Cl.: A61L 29/08, A61L 29/16, A61M 25/10

(54) **DRUG COATING, DRUG COATING SACCULE AND METHOD FOR PREPARING SAME**
ARZNEIMITTELBESCHICHTUNG, ARZNEIMITTELBESCHICHTUNGSSACK UND VERFAHREN ZUR HERSTELLUNG DAVON
ENROBAGE DE MÉDICAMENT, SACCULE D'ENROBAGE DE MÉDICAMENT ET SON PROCÉDÉ DE PRÉPARATION

(43) Date of publication of application: 14.02.2024
(73) Proprietor: LEPU MEDICAL TECHNOLOGY (BEIJING) CO., LTD., Beijing 102200 (CN)
(72) Inventor: LIU, Yangyang, Beijing 102200 (CN); ZHAO, Lixiao, Beijing 102200 (CN); MA, Cuijie, Beijing 102200 (CN); ZHANG, Yi, Beijing 102200 (CN); HU, Xiaojun, Beijing 102200 (CN)
(74) Representative: J A Kemp LLP
(86) International application number: PCT/CN2022/098130
(87) International publication number: WO 2023/236188

(56) References cited:
- WO-A1-2022/105849
- CN-A- 107 073 178
- CN-A- 107 206 129
- CN-A- 110 496 251
- CN-A- 111 001 044
- CN-A- 111 317 907
- CN-A- 111 672 018
- CN-A- 111 973 813
- CN-B- 103 830 778
- US-A1- 2015 273 117
- US-B2- 10 617 793
- CHEN WEIPING, ZHAN HONG-BING, LI ZHAO-HUI: "Drug Loading Strategies and Applications of Drug Eluting Stents", CHINESE JOURNAL OF TISSUE ENGINEERING RESEARCH, ZHONGGUO KANGFU YIXUEHUI,, CN, vol. 14, no. 42, 15 October 2010 (2010-10-15), CN , pages 7878 - 7882, XP093046379, ISSN: 1673-8225, DOI: 10.3969/j.issn.1673-8225.2010.42.021

## Description

### TECHNICAL FIELD

The present invention relates to the technical field of medical devices, in particular to a drug-coated balloon and a preparation method thereof.

### BACKGROUND

Coronary heart disease is the most common cause of death in the world today. The deaths number caused by coronary heart disease even exceeds the total death number caused by all kinds of cancers. According to incomplete statistics, the number of coronary heart disease patients in China has exceeded 11 million, and the number is increasing by 1 million every year. There were more than 900,000 percutaneous coronary intervention (PCI) operations in China in 2018. It is expected that PCI will continue to maintain an annual rate of 13%-16% growth in the next 3-5 years against a backdrop of younger age of coronary heart disease, an aging population and the continuous advancement of tiered diagnosis and treatment. Drug-coated balloon (DCB, referred to as drug balloon) is a coronary intervention technology developed in recent years. The surface of the drug balloon is coated with a drug coating, which is transferred to the blood vessel wall within a short dilation time after being delivered to the lesion site, and the drug coating releases anti-proliferative drugs to inhibit the proliferation of vascular smooth muscle cells. Drugs such as rapamycin are safe in the human body, but due to their poor lipophilicity, slow tissue absorption, and low bioavailability, the above shortcomings need to be overcome when applied to the field of drug balloons. WO 2022/105849 A1 discloses a drug-coated balloon and a preparation method therefor, the drug-coated balloon comprising a balloon body and a drug coating arranged on an outer surface of the balloon body, and the drug coating comprising a sequentially stacked bottom coating layer, interlayer coating layer, and top coating layer. The bottom coating layer is used for increasing the firmness and adhesion of the drug coating to the balloon body, reducing drug loss during delivery and increasing the rate of utilisation of the drug: the interlayer coating laver is used for loading the drug and also helps the drug to be better absorbed by the tissue; and the top coating laver is used for promoting the adhesion between the drug coating layer and the inner membrane of the vascular wall to better facilitate the tissue absorption capacity of the drug.

However, the current drug balloons mainly have the following problems: a large amount of drug is lost during the delivery process, and the blood wash or the dissolution and shedding of the drug coating causes a large loss of the drug coating on the surface of the balloon, thus affecting the drug amount transferred from the drug coating to the blood vessel wall of the lesions. According to research, 80% or more of the drugs in the drug-coated capsules in the prior art are lost in the drug delivery process, reducing the drug amount transferred to the blood vessel wall, thereby reducing the efficacy; These lost drugs may enter the human circulatory system and increase the risk of downstream vascular embolism, and increase the toxic and side effects of drug-coated balloons. Therefore, how to reduce the amount of drug delivered and increase the amount of drug transferred to the vascular wall to improve the efficacy is still an urgent problem for researchers to solve.

### SUMMARY OF THE INVENTION

Therefore, the technical problem to be solved by the present invention is to overcome the defect of low drug amount transferred from the drug balloon to blood vessel wall in the prior art, thereby providing a novel drug-coated balloon and a preparation method thereof.

To this end, the present invention provides a drug-coated balloon comprising a balloon body and a drug coating attached to the outside of the balloon body, wherein the drug coating comprises a drug active coating and a positively-charged hydrophobic modified layer which are sequentially attached to the surface of a substrate, and the positively-charged hydrophobic modified layer comprises a positively-charged modified substance and a hydrophobic substance, the drug active coating comprises a core-shell structure layer and a drug nanoparticle layer, the hydrophobic substance is phytanic acid and the positively-charged modified substance is dioleoyl phosphatidylethanolamine.

In some embodiments, the core-shell structure layer comprises a core-shell structure particle, the core-shell structure particle has an inner core and an outer shell surrounding the inner core, the inner core is a drug particle, and the outer shell is a polymer shell; and the drug nanoparticle layer comprises a drug nanoparticle.

In some embodiments, the core-shell structure particle has a particle size D50 of 100 nm to 9 µm; preferably 300 nm to 6 µm.

In some embodiments, the drug nanoparticle has a particle size D50 of 100nm-600nm.

In some embodiments, the drug coating comprises a core-shell structure layer, a drug nanoparticle layer and a positively-charged hydrophobic modified layer which are sequentially attached to the surface of the substrate.

In some embodiments, the drug coating has at least one feature selected from the group consisting of the following A-F:
A, the core-shell structure layer comprises a binder, preferably, the binder is at least one selected from the group consisting of polyvinyl alcohol, polyvinylpyrrolidone, Tween 80, poloxamer, egg yolk lecithin, soybean lecithin and methyl cellulose, preferably, a mass ratio of the binder to the core-shell structure particle is 1: 1-26;
B, the polymer shell is an amphiphilic polymer shell, and the amphiphilic polymer comprises a hydrophilic block and a hydrophobic block, wherein the hydrophilic block is polyethylene glycol or polyethylene glycol monomethyl ether, and the hydrophobic block is at least one selected from the group consisting of polyoxypropylene, polystyrene, polyamino acid, polyglycolic acid, polylactide, polycaprolactone and poly(lactic-co glycolic acid); preferably, a mass ratio of the drug particle to the polymer shell in the core-shell structure particle is 0.5-5:0.5-50;
C, the drug nanoparticle layer comprises a binder, preferably, the binder is at least one selected from the group consisting of polyvinyl alcohol, polyvinylpyrrolidone, Tween 80, poloxamer, egg yolk lecithin, soybean lecithin and methyl cellulose; preferably, a mass ratio of the binder to the drug nanoparticle is 1:1-25;
D, a ratio of the drug content in the core-shell structure layer to the drug content in the drug nanoparticle layer is 1-5:1-5; preferably 1-2:1-2;
E, the drug is a medicine for preventing and treating coronary heart disease, preferably, the medicine for preventing and treating coronary heart disease is selected from rapamycin and/or a rapamycin derivative; and
F, the drug content in the core-shell structure layer is 1-5 µg/mm²; and/or the drug content in the drug nanoparticle layer is 1-5 µg/mm².

Wherein, the polyamino acid in the present invention includes common polyamino acids such as polyglutamic acid, polyaspartic acid, and polyomithine.

In some embodiments, the drug nanoparticle layer is also provided with a positively-charged hydrophobic modified layer on the side away from the core-shell structure layer. Preferably, the positively-charged hydrophobic modified layer comprises a positively-charged lipid substance and a hydrophobic substance; more preferably, the positively-charged hydrophobic modified layer has at least one feature selected from the group consisting of following (1) to (3):
(1) a mass ratio of the positively-charged substance to the hydrophobic substance is 10-20:10-20, preferably 20:10;
(2) based on the total mass of the positively-charged modified substance and the hydrophobic substance, the content of the positively-charged hydrophobic modified layer is 0.1-1 µg/mm².

In some embodiments, the core-shell structure particle comprises the following raw materials: 0.5-5 parts by weight of a drug, 0.5-50 parts by weight of an amphiphilic polymer, 1-50 parts by volume of an oil phase, 100-2000 parts by volume of an aqueous solution containing an emulsifier; wherein, the proportioning relationship between parts by weight and parts by volume is g/mL; optionally, the oil phase is selected from dichloromethane, acetone and any combination thereof.

In some embodiments, the drug nanoparticle comprises the following raw materials: 0.5-2 parts by weight of a drug, 5-20 parts by volume of an oil phase, and 5-100 parts by volume of an aqueous solution containing an emulsifier; wherein, the proportioning relationship between parts by weight and parts by volume is g/mL; optionally, the oil phase is selected from methanol, ethanol and any combination thereof.

In some embodiments, the emulsifier is at least one selected from the group consisting of polyvinyl alcohol, polyvinylpyrrolidone, poloxamer, bovine serum albumin, Tween 80, poloxamer, egg yolk lecithin, soybean lecithin and methyl cellulose; and/or, the emulsifier is present in an amount of 0.01-2% (weight/volume) in the aqueous solution containing the emulsifier.

The present invention also provides a method for preparing a drug-coated balloon, comprising the following steps:
step S1: spraying a drug-containing coating solution on the outside of the balloon body, and drying to form a drug active coating;
step S2: mixing a positively-charged modified substance, a hydrophobic substance and an organic solvent to prepare a coating solution of the positively-charged hydrophobic modified layer, spraying the coating solution of the positively-charged hydrophobic modified layer on the outside of the drug active coating, and drying to form the positively-charged hydrophobic modified layer, obtaining a drug-coated balloon.

In the present invention, the drying method for forming the coating may be common drying such as airing and stoving.

In some embodiments, the organic solvent is at least one selected from the group consisting of alkanes, alcohols and water; and/or a ratio of the positively-charged modified substance by mass to the hydrophobic substance by mass to the organic solvent by volume is 10-20mg:10-20mg:5mL. The exemplary organic solvent is at least one selected from n-heptane, n-hexane, n-pentane, methanol, ethanol and water.

In some embodiments, the drug-containing coating solution comprises a coating solution of the core-shell structure layer and/or a coating solution of the drug nanoparticle layer;
wherein the coating solution of the core-shell structure layer is prepared by: mixing the core-shell structure particle with the organic solvent to obtain the coating solution of the core-shell structure layer; and
wherein the coating solution of the drug nanoparticle layer is prepared by: mixing the drug nanoparticle with the organic solvent to obtain the coating solution of the drug nanoparticle layer.

In some embodiments, the organic solvent is at least one selected from the group consisting of alkanes, alcohols and water; and/or, in the coating solution of the core-shell structure layer, a ratio of the added core-shell structure particle by mass to the organic solvent by volume is 2.1g:3-12mL; and/or, in the coating solution of the drug nanoparticle layer, a ratio of the added drug nanoparticle by mass to the organic solvent by volume is 150mg:3-12mL. The exemplary of the organic solvent is at least one selected from n-heptane, n-hexane, n-pentane, methanol, ethanol and water.

The core-shell structure particle in the present invention can be prepared by conventional methods. An exemplary preparation is as follows: the drug and the amphiphilic polymer can be dissolved in an oil phase, then the oil phase is added dropwise to an aqueous solution containing an emulsifier, followed by performing at least one or a combination of high pressure homogenization, ultrasonic homogenization, solvent evaporation and membrane emulsification, then centrifugation, removal of supernatant, and drying to obtain the core-shell structure particle.

The drug nanoparticle in the present invention can be prepared by conventional methods.

An exemplary preparation is as follows: the drug can be dissolved in an oil phase, then the oil phase is added dropwise to an aqueous solution containing an emulsifier, stirred or sonicated, and dried to obtain the drug nanoparticle.

The core-shell structure particle and the drug nanoparticle can be dried by conventional methods such as spray drying, freeze drying and vacuum drying. One or two lyoprotectants are added before freeze drying, types of which include mannitol, trehalose, sucrose, glucose, lactose, etc., and the lyoprotectant can be present in an amount of 0.1%-10% or 1-10% by mass in the drug.

The technical solution of the present invention has the following advantages:
1. The drug coating according to the present invention comprises a drug active coating and a positively-charged hydrophobic modified layer which are sequentially attached to the surface of a substrate, and the positively-charged hydrophobic modified layer comprises a positively-charged modified substance and a hydrophobic substance. By providing the positively charged hydrophobic modified layer comprising the positively-charged modified substance and the hydrophobic substance, the entire surface of the drug coating is given positive electric charge and hydrophobic property. Such positive electric charge characteristics make the drug coating can be strongly combined with the negatively-charged inner wall of blood vessels, so that the drug coating can be permanently adsorbed on the inner wall of blood vessels. The combination of positive electric charge characteristics and hydrophobic property can effectively resist the influence of blood flow on the erosion of drug coating during transportation. The drug coating with the above structure can not only reduce the loss of the drug during the delivery of the drug balloon, but also reduce the loss of the drug during balloon dilation and contraction, while increasing the amount of drug transferred to the inner wall of the blood vessel.
2. The drug coating according to the present invention is provided with the drug nanoparticle and/or the core-shell structure layer, in particular, the drug coating comprising a core-shell structure layer, a drug nanoparticle layer and a positively-charged hydrophobic modified layer which are sequentially attached to the surface of the substrate. On the one hand, this feature makes the drug nanoparticles more firmly bound on the surface of the balloon, not easy to fall off, thereby greatly reducing the amount of drug loss in the process of delivery in the body, increasing the amount of drug transferred to the blood vessel wall, and thus improving the curative effect; on the other hand, the drug nanoparticle is arranged in the drug nanoparticle layer and the drug particle is arranged in the core-shell structure layer, so that drugs in the drug nanoparticle layer can be released quickly after reaching the affected area to achieve the purpose of rapid onset, while drugs in the core-shell structure layer is slowly released and can achieve a sustained release effect and prolong the action time. Furthermore, it is possible to regulate the drug release by adjusting the drug content in the core-shell structure layer and the drug nanoparticle layer.
3. In the drug coating according to the present invention, the particle size D50 of the core-shell structure particles is controlled in a range of 100 nm and 9 µm to regulate the drug to have a reasonable release period, and maintain the drug concentration in the target tissue at a relatively high therapeutic level. Especially controlling within a range of 300 nm to 6 µm allows the drug to be released in about 90 days.
4. In the drug coating according to the present invention, the particle size of the drug nanoparticle is 100-600 nm, and the nano-scale drug particles can quickly penetrate into the target diseased tissue and act immediately.
5. In the drug coating provided in the present invention, the core-shell structure layer further comprises a binder which preferably is at least one selected from the group consiting of polyvinyl alcohol, polyvinylpyrrolidone, Tween 80, poloxamer, egg yolk lecithin, soybean lecithin and methyl cellulose; or the drug nanoparticle layer further comprises a binder which preferably is at least one selected from the group consisting of polyvinyl alcohol, polyvinylpyrrolidone, Tween 80, poloxamer, egg yolk lecithin, soybean lecithin and methyl cellulose; the addition of the binder increases the cohesive force between the core-shell particles, resulting in lower coating loss during transportation and dilation, increasing the affinity of the coating with the blood vessel wall, making the coating transfer higher; increasing the lipophilicity of the drug, allowing the drug to enter the target tissue faster.
6. The drug coating according to the present invention can further reduce the loss rate and increase the amount transferred to vessel wall by adopting the hydrophobic substances and positively-charged modified substances listed in the present invention, especially by sslecting phytanic acid as the hydrophobic substance and dioleoyl phosphatidylethanolamine as the positively-charged modified substance.

### BRIEF DESCRIPTION OF THE DRAWINGS

In order to illustrate the specific embodiments of the present invention or the technical solutions in the prior art more clearly, the following briefly introduces the accompanying drawings that need to be used in the description of the specific embodiments or the prior art.

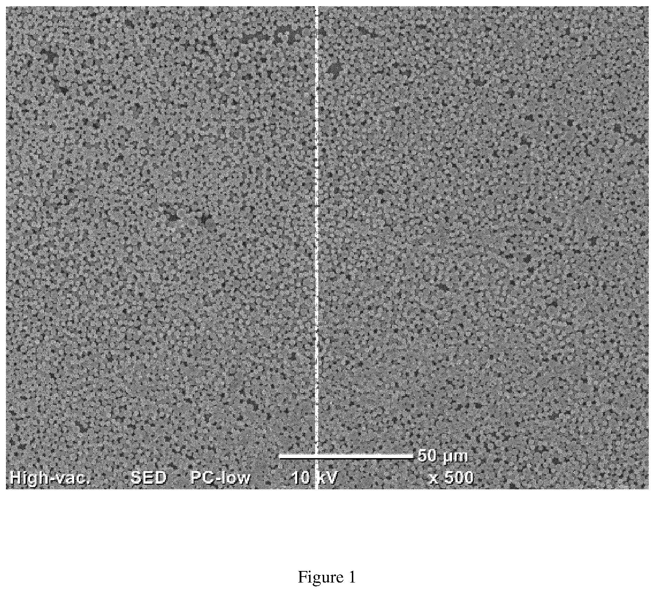

Figure 1 is a scanning electron microscope image of the core-shell structure particle prepared in Reference Example 1 of the present invention;

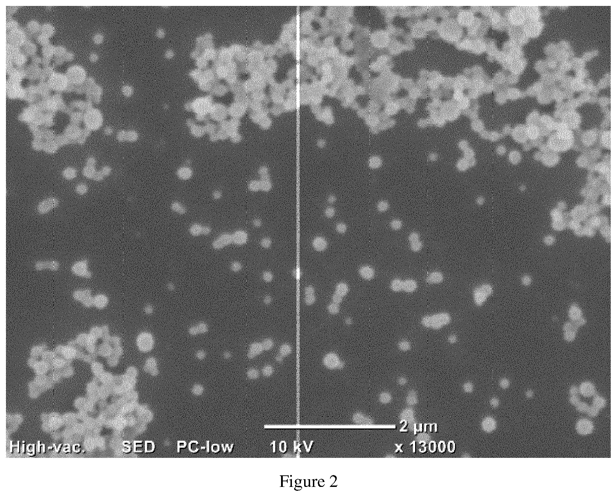

Figure 2 is a scanning electron microscope image of the drug nanoparticle prepared in Reference Example 1 of the present invention;

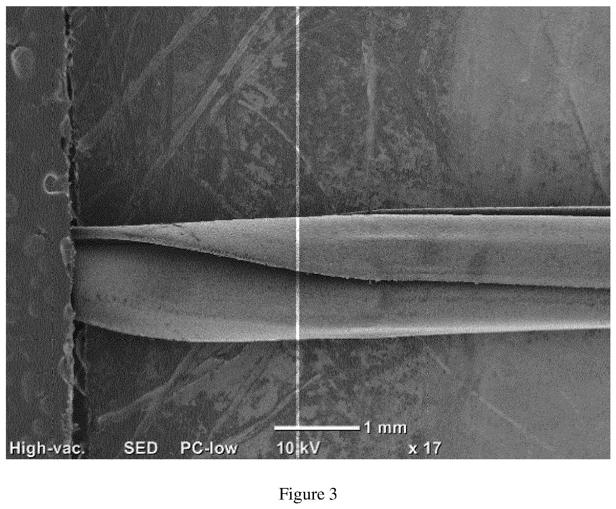

Figure 3 is a scanning electron microscope image of the drug balloon prepared in Reference Example 1 of the present invention.

### DETAILED DESCRIPTION

If the specific experimental steps or conditions are not indicated in the examples, it can be carried out according to the operations or conditions of the conventional experimental steps described in the literature in this field. The reagents or instruments used without the manufacturer's indication are all conventional reagent products that can be obtained from commercial sources.

For example, the polyethylene glycol-polycaprolactone block copolymer (PEG-PCL) used in the present invention was purchased from SHANGHAI ZZBIO CO., LTD., PEG number average molecular weight: 2000, PCL number average molecular weight: 2000. Polyethylene glycol-polylactide block copolymer (PEG-PLA) was purchased from SHANGHAI ZZBIO CO., LTD., PEG number average molecular weight: 2000, PLA viscosity: 0.9-1.2 dl/g. Polyethylene glycol-polyglycolide lactide block copolymer (PEG-PLGA), PEG number average molecular weight: 2000, PLGA (75:25) viscosity: 0.5-0.8dl/g, PLGA (50:50) viscosity: 0.3-0.5dl/g, all purchased from: XI'AN RUIXI BIOLOGICAL TECHNOLOGY CO., LTD.

Polyvinyl alcohol (PVA) was purchased from GUANGZHOU STANDARD PHARMA LTD., model: 4-88; Sodium alginate was purchased from QINGDAO HUANGHAI BIO-PHARMACEUTICAL CO., LTD.; Trehalose was purchased from JIANGSU HI-STONE PHARMACEUTICAL CO., LTD., Specification: injection grade; Poloxamer 188, was purchased from SIGMA-ALDRICH (SHANGHAI) TRADING CO., LTD.; Lactose was purchased from JIANGSU HI-STONE PHARMACEUTICAL CO., LTD. Specification: injection grade. The egg yolk lecithin was purchased from Lipoid Company, specification: injection grade; the balloon was provided by LEPU MEDICAL TECHNOLOGY (BEIJING) CO., LTD., 3.5mm diameter×20mm length.

The method for testing the drug loading of the core-shell structured particles in the present invention is as follows: 50 mg of the core-shell structured particles is mixed with 5 mL of acetonitrile, sonicated for 10 minutes, diluted with acetonitrile to 10 mL, and centrifuged. The supernatant is collected and injected to a high performance liquid chromatograph respectively to determine the content of the drug (rapamycin) in core-shell structured particles according to the Pharmacopoeia of the People's Republic of China (2020 edition) general rule 0512 high performance liquid chromatography, with octadecylsilane bonded silica gel as a filler, methanol-acetonitrile-water (60:17:23) as a mobile phase, under a detection wavelength of 280 nm, at a flow rate of 1 mL/min, and a column temperature of 40°C. Particle size D50 test: 10 mg of core-shell structured particles or drug nanoparticles is dispersed well in 5 ml of purified water, and then tested with a particle size distribution analyzer; the average value of three consecutive measurements is taken.

### REFERENCE EXAMPLE 1

A drug-coated balloon was provided in this embodiment. The drug-coated balloon comprised a balloon body, and a core-shell structure layer, a drug nanoparticle layer and a positively-charged hydrophobic modified layer which were sequentially attached to the outside of the balloon body, wherein the core-shell structure layer comprised a core-shell structure particle, the core-shell structure particle had an inner core and an outer shell surrounding the inner core, the inner core was a rapamycin particle, and the outer shell was a PEG-PCL shell; the drug nanoparticle layer comprised rapamycin nanoparticles as drug nanoparticles. The positively-charged hydrophobic modified layer comprised a positively-charged modified substance (DC-cholesterol) and a hydrophobic modified substance (stearic acid).

The preparation method of the above-mentioned drug-coated balloon comprised the following steps:
(1)Preparation of Core-Shell Structure Particles
   1g of rapamycin and 2g of PEG-PCL were weighed and dissolved in 30mL of an oil phase (dichloromethane and acetone at a volume ratio of 8:2). Under stirring (normal temperature, 1000r/min), 200ml of purified water containing 0.1% (w/v) PVA was dropwise (1mL/min) injected, stirred for 12h (35°C, 750r/min), and centrifuged at -4°C and 12000r/min for 30min. The supernatant was removed, and the bottom precipitate was washed for twice with 200 ml of purified water each time, centrifuged, and freeze-dried to obtain core-shell structure particles. Test showed that the core-shell particles had a particle diameter D50 of 312 nm, and a drug loading of 6.83%.
(2)Preparation of Drug Nanoparticles
   Rapamycin drug nanoparticles were prepared by anti-solvent precipitation method. 0.5 g of rapamycin was weighed and dissolved in 5 ml of methanol to obtain an oil phase. The oil phase was added dropwise to 15 ml of an aqueous solution containing 2% (w/v) of poloxamer 188 and sonicated for 30 min, then 25 mg of trehalose was added thereto and dispersed well, then freeze-dried to obtain drug nanoparticles. After testing, the particle size D50 of the drug nanoparticles was 481 nm.
(3)Preparation of Coating Solution
   2.1 g of core-shell structure particles and 83.6 mg of egg yolk lecithin were added to 12 ml of ethanol and dispersed well by ultrasonic to obtain a coating solution of core-shell structure layer. 150 mg of drug nanoparticles and 7.1 mg of egg yolk lecithin were added to 12 ml of ethanol and dispersed well by ultrasonic to obtain a coating solution of drug nanoparticle layer. 20 mg of DC-cholesterol and 20 mg of stearic acid were added into 5 ml of ethanol and dispersed well by ultrasonic to obtain a coating solution of a positively-charged hydrophobic modified layer.
(4)Preparation of Drug Balloon
   The first spraying: the coating solution of the core-shell structure layer was sprayed on the surface of the balloon by using a balloon spraying machine at following spraying parameters of: a dripping flow rate: 3.6ml/h, an ultrasonic power: 0.05W, so that the concentration of rapamycin on the surface of the balloon reached 1.0 µg/mm², and it was air-dried;
   The second spraying: the coating solution of the drug nanoparticle layer was sprayed on the surface of the balloon by using a balloon spraying machine at following spraying parameters of: a dripping flow rate: 3.6ml/h, an ultrasonic power: 0.05W, so that the concentration of rapamycin on the surface of the balloon reached 2.0 µg/mm², and it was air-dried;
   The third spraying: the coating solution of the positively-charged hydrophobic modified layer was sprayed on the surface of the balloon by using a balloon spraying machine at following spraying parameters of: drip flow rate: 3.6ml/h, ultrasonic power: 0.05W, and it was air-dried. The balloon after the third spraying had an average weight gain of 0.2 µg/mm². The average weight gain of the third spraying balloon in the present invention was calculated as follows: the average weight gain of the third spraying balloon = (the weight of the balloon after the third spraying and drying - the weight of the balloon after the second spraying and drying)/balloon surface area.

### REFERENCE EXAMPLE 2

A drug-coated balloon and a preparation method thereof provided in this embodiment differed from Reference Example 1 only in that the composition and preparation method of the coating solution of the positively-charged hydrophobic modified layer were different. In this embodiment, 20 mg of DC-cholesterol and 10 mg of stearic acid were added to 5 ml of ethanol and dispersed well by ultrasonic to obtain a coating solution of a positively-charged hydrophobic modified layer.

### REFERENCE EXAMPLE 3

A drug-coated balloon and a preparation method thereof provided in this embodiment differed from Reference Example 1 only in that the average weight gain of the balloon after the third spraying was 0.1 µg/mm².

### EXAMPLE 4

A drug-coated balloon and a preparation method thereof provided in this embodiment differed from Reference Example 1 only in that the composition and preparation method of the coating solution of the positively-charged hydrophobic modified layer were different and the weight gain by spraying was different. In this embodiment, 20 mg of dioleoyl phosphatidylethanolamine (DOPE) and 20 mg of phytanic acid were added into 5 ml of ethanol and dispersed well by ultrasonic to obtain a coating solution of a positively-charged hydrophobic modified layer for later use. In this embodiment, the average weight gain of the balloon after the third spraying was 0.1 µg/mm².

### EXAMPLE 5

A drug-coated balloon and a preparation method thereof provided in this embodiment differed from Reference Example 1 only in that the composition and preparation method of the coating solution of the positively-charged hydrophobic modified layer were different. In this embodiment, 20 mg of DOPE and 10 mg of phytanic acid were added to 5 ml of ethanol and dispersed well by ultrasonic to obtain a coating solution of a positively-charged hydrophobic modified layer for later use.

### EXAMPLE 6

A drug-coated balloon and a preparation method thereof provided in this embodiment differed from Reference Example 1 only in that the composition and preparation method of the coating solution of the positively-charged hydrophobic modified layer were different. In this embodiment, 20 mg of DOPE and 20 mg of phytanic acid were added to 5 ml of ethanol and dispersed well by ultrasonic to obtain a coating solution of a positively-charged hydrophobic modified layer for later use.

### EXAMPLE 7

A drug-coated balloon and a preparation method thereof provided in this embodiment differed from Reference Example 1 only in that the preparation methods of the coating solution of the core-shell structure layer and the coating solution of the drug nanoparticle layer were different. In this embodiment, 2.1 g of the core-shell structure particles were added into 12 ml of ethanol and dispersed well by ultrasonic to obtain a coating solution of the core-shell structure layer. 150 mg drug nanoparticles were added into 12ml of ethanol and dispersed well by ultrasonic to obtain a coating solution of the drug nanoparticle layer.

### EXAMPLE 8

A drug-coated balloon was provided in this embodiment. The drug-coated balloon comprised a balloon body, and a core-shell structure layer, a drug nanoparticle layer and a positively-charged hydrophobic modified layer which were sequentially attached to the outside of the balloon body, wherein the core-shell structure layer comprised a core-shell structure particle, the core-shell structure particle had an inner core and an outer shell surrounding the inner core, the inner core was a rapamycin particle, and the outer shell was a PEG-PLA shell; the drug nanoparticle layer comprised rapamycin nanoparticles as drug nanoparticles. The positively-charged hydrophobic modified layer comprised DC-cholesterol and stearic acid.

The preparation method of the above-mentioned drug-coated balloon comprised the following steps:
(1)Preparation of Core-Shell Structure Particles
   0.5 g of rapamycin and 0.5 g of PEG-PLA were weighed and dissolved in 1mL of oil phase (acetone). Under stirring (normal temperature, 1000r/min), 100ml of purified water containing 0.1% (w/v) PVA was dropwise (1mL/min) injected, stirred for 12 h (35°C, 750r/min), centrifuged at -4°C and 12000r/min for 30 min. The supernatant was removed, and the bottom precipitate was washed for twice with 200 ml of purified water each time, centrifuged, and freeze-dried to obtain core-shell structure particles. Test showed that the core-shell particles had a particle diameter D50 of was 579 nm and a drug loading of 5.92%.
(2)Preparation of Drug Nanoparticles
   Rapamycin drug nanoparticles were prepared by anti-solvent precipitation method. 2 g of rapamycin was weighed and dissolved in 20 ml of ethanol to obtain an oil phase. The oil phase was added dropwise to 100 ml of an aqueous solution containing 2% (w/v) of poloxamer 188 and sonicated for 30 min, then 25 mg of mannitol was added thereto and dispersed well, then freeze-dried to obtain drug nanoparticles. After testing, the particle size D50 of the drug nanoparticles was 593 nm.
(3)Preparation of Coating Solution
   2.1 g of core-shell structure particles and 2.1 g of Twain 80 were added to 3 ml of water and dispersed well by ultrasonic to obtain a coating solution of core-shell structure layer. 150 mg of drug nanoparticles and 150 mg of poloxamer 188 were added to 3 ml of water and dispersed well by ultrasonic to obtain a coating solution of drug nanoparticle layer. 20 mg of DC-cholesterol and 20 mg of stearic acid were added into 5 ml of n-hexane and dispersed well by ultrasonic to obtain a coating solution of a positively-charged hydrophobic modified layer.
(4)Preparation of Drug Balloon
   The first spraying: the coating solution of the core-shell structure layer was sprayed on the surface of the balloon by using a balloon spraying machine at following spraying parameters of: the dripping flow rate: 3.6ml/h, the ultrasonic power: 0.05W, so that the concentration of rapamycin on the surface of the balloon reached 1.0 µg/mm², and it was air-dried;
   The second spraying: the coating solution of the drug nanoparticle layer was sprayed on the surface of the balloon by using a balloon spraying machine at following spraying parameters of: the dripping flow rate: 3.6ml/h, the ultrasonic power: 0.05W, so that the concentration of rapamycin on the surface of the balloon reached 2.0 µg/mm², and it was air-dried;
   The third spraying: the coating solution of the positively-charged hydrophobic modified layer was sprayed on the surface of the balloon by using a balloon spraying machine at following spraying parameters of: drip flow rate: 3.6ml/h, ultrasonic power: 0.05W, and it was air-dried. The balloon after the third spraying had an average weight gain of 0.2 µg/mm².

### EXAMPLE 9

A drug-coated balloon and a preparation method thereof were provided in this embodiment. The drug nanoparticles, the coating solution of drug nanoparticle layer and the coating solution of positively-charged hydrophobic modification layer were prepared according to the methods of Example 1.

The first spraying: the coating solution of the drug nanoparticle layer was sprayed on the surface of the balloon by using a balloon spraying machine at following spraying parameters: the dripping flow rate: 3.6ml/h, the ultrasonic power: 0.05W, so that the concentration of rapamycin on the surface of the balloon reached 2.0 µg/mm², and it was air-dried;
The second spraying: the coating solution of the positively-charged hydrophobic modified layer was sprayed on the surface of the balloon by using a balloon spraying machine at following spraying parameters: drip flow rate: 3.6ml/h, ultrasonic power: 0.05W, and it was air-dried. The balloon after the second spraying had an average weight gain of 0.2 µg/mm². The average weight gain of the balloon after the second spraying in the present invention was calculated as follows: the average weight gain of the second spraying balloon = (the weight of the balloon after the second spraying and drying - the weight of the balloon after the first spraying and drying)/balloon surface area.

### EXAMPLE 10

A drug-coated balloon and a preparation method thereof were provided in this embodiment. The core-shell structure particles, the coating solution of the core-shell structure layer and the coating solution of positively-charged hydrophobic modification layer were prepared according to the method of Example 1.

The first spraying: the coating solution of the core-shell structure layer was sprayed on the surface of the balloon by using a balloon spraying machine at following spraying parameters of: the dripping flow rate: 3.6ml/h, the ultrasonic power: 0.05W, so that the concentration of rapamycin on the surface of the balloon reached 2.0 µg/mm², and it was air-dried;
The second spraying: the coating solution of the positively-charged hydrophobic modified layer was sprayed on the surface of the balloon by using a balloon spraying machine at following spraying parameters: drip flow rate: 3.6ml/h, ultrasonic power: 0.05W, and it was air-dried. The balloon after the second spraying had an average weight gain of 0.2 µg/mm².

### COMPARATIVE EXAMPLE 1

A drug-coated balloon was provided in this comparative example. The drug-coated balloon comprised a balloon body, and a core-shell structure layer and a drug nanoparticle layer which were sequentially attached to the outside of the balloon body, wherein the core-shell structure layer comprised a core-shell structure particle, the core-shell structure particle had an inner core and an outer shell surrounding the inner core, the inner core was a rapamycin particle, and the outer shell was a PEG-PCL shell; the drug nanoparticle layer comprised rapamycin nanoparticles as drug nanoparticles.

The above-mentioned drug-coated balloon was prepared according to the method of Example 1, except that the surface of the balloon was not sprayed for the third time, that is, no coating solution of the positively-charged hydrophobic modified layer was sprayed.

### EXPERIMENTAL EXAMPLE 1 Investigating the in vitro drug release of core-shell structure particles with different particle sizes

To investigate the *in vitro* release of core-shell structure particles with different particle sizes, 5 groups of experiments were done. The mass of rapamycin and PEG-PCL, the volume of oil phase, and the volume ratio of dichloromethane to acetone in the oil phase and the stirring speed for each group of experiments are shown in the following Table 1. Rapamycin and PEG-PCL according to the following table prescription were weighed respectively, dichloromethane and acetone were weighed according to the prescription table below and mixed to prepare the oil phase, then rapamycin and PEG-PCL were dissolved in the oil phase, resulting in a drug-containing oil phase. Then the drug-containing oil phase was stirred at the corresponding rotation speed in the table at normal temperature, and 200 ml of an aqueous solution containing 0.1% (w/v) PVA was dropwise (1 mL/min) injected under stirring. After injecting was completed, the stirring was continued at 750 r/min for 12 h at a temperature of 35°C. Then, centrifugation was carried out at a rotating speed of 12000 r/min and a temperature of -4°C for 30min, the supernatant was removed, the bottom precipitate was washed twice with purified water, 200 ml of water each time, centrifuged, and freeze-dried to obtain a core-shell structure particle.

**Table 1 Core-shell structure particles and particle sizes thereof obtained with different formulations and process conditions**

| Experimental Group No. | Rapamycin /g | PEG-PCL/g | Oil Phase /mL | Volume ratio of Methylene Chloride to Acetone in the Oil Phase | Speed of Stirring/rpm | Particle Size D50 |
|---|---|---|---|---|---|---|
| Experimental Group 1 | 0.5 | 1.0 | 50 | 8: 2 | 1000 | 108nm |
| Experimental Group 2 | 1.0 | 2.0 | 30 | 8: 2 | 700 | 341nm |
| Experimental Group 3 | 1.0 | 2.0 | 15 | 8: 2 | 600 | 2.59µm |
| Experimental Group 4 | 1.0 | 3.0 | 15 | 8: 2 | 600 | 5.94µm |
| Experimental Group 5 | 1.0 | 4.0 | 10 | 8: 2 | 400 | 8.73µm |

Then the *in vitro* drug release of the core-shell structure particles was tested by the following method.

10 mg of core-shell structure particles were mixed with an appropriate amount of a release medium, shaken to disperse well, fully transferred into a 1000ml volumetric flask, diluted with the release medium to the mark, and shaken to mix well. Shake flask method was carried out as follows: 160 rpm/min, release medium was PBS (pH7.4) solution containing 0.03% (w/v) SDS, volume was 40ml; samples were taken at: 1 day, 4 days, 7 days, 14 days, 28 days, 60 days and 90 days respectively, 1.5ml for each sample, and 1.5ml replenishment after taking of each sample. The solution to be tested was passed through a 0.5um filter membrane for later use. The content of rapamycin in the test solution was determined according to General Chapter 0512 of the Pharmacopoeia of the People's Republic of China (2020 edition), using octadecylsilane-bonded silica gel as the filler, methanol-acetonitrile-water (at a volume ratio of 60:17:23) as the mobile phase, under a detection wavelength of 280 nm, with a flow rate of 1 mL/min and a column temperature of 40 °C.

**Table 2 Cumulative release (%)**

| Sampling Time (Day) | Experimental Group 1 | Experimental Group 2 | Experimental Group 3 | Experimental Group 4 | Experimental Group 5 |
|---|---|---|---|---|---|
| 1 | 30.2 | 13.2 | 18.8 | 14.3 | 15.8 |
| 4 | 37.9 | 30.2 | 28.4 | 20.1 | 18.3 |
| 7 | 49.3 | 40.8 | 39.3 | 25.4 | 20.1 |
| 14 | 72.1 | 62.4 | 49.2 | 30.2 | 25.4 |
| 28 | 100 | 78.2 | 58.3 | 41.5 | 30.9 |
| 60 | No Test | 91.6 | 88.6 | 70.4 | 57.2 |
| 90 | No Test | 100 | 100 | 84.2 | 69.9 |

It can be seen from the above table that, compared with the experimental group 1, the experimental groups 2-4 had better sustained-release effect, and the effect of prolonging the action time of the drug was better. However, in test group 1, the drug release was faster due to the smaller particle size, and 100% of the drug was released within 28 days, while the drug release cycle in test group 5 was too long due to the larger particle size, and only 69.9% was released in 90 days. In the present invention, the particle size D50 of the core-shell structure particles was preferably 300 nm to 6 µm.

### EXPERIMENTAL EXAMPLE 2

In vitro simulation transport equipment (refer to the pharmaceutical industry standard "YY/T 0807-2010") was used to simulate *in vivo* delivery withdrawal and dilation of the drug balloons prepared by each example and the comparative example to study drug loss during delivery, withdrawal and dilation and the drug coating transferring to the inner wall of the blood vessel during dilation, thereby indirectly evaluating the effectiveness, safety and anti-scour ability of the coating.

Test method: The *in vitro* simulated transport equipment was heated in a water bath and maintained at a temperature of 37°C. The end of the equipment was connected to a silicone simulated blood vessel (provided by Preclinic Medtech (Shanghai) Co., Ltd., diameter 3.0 mm). The drug balloon product was inserted into this equipment. When the balloon body entered the simulated blood vessel, the balloon was inflated to a pressure of 8 atm. After maintaining the pressure for 1 min, negative pressure was applied. Liquids in the simulated blood vessel, the simulated blood vessel, liquids in the *in vitro* simulated transportation equipment and the balloon after dilation were collected in turn. The content of rapamycin therein was tested according to the following method. The sample to be tested was dissolved in solvent acetonitrile, quantitatively diluted, and centrifuged. The supernatant was taken and injected into a liquid chromatograph. The content of rapamycin in the test solution was determined according to General Chapter 0512 of the Pharmacopoeia of the People's Republic of China (2020 edition), using octadecylsilane-bonded silica gel as the filler, methanol-acetonitrile-water (at a volume ratio of 60:17:23) as the mobile phase, under the detection wavelength of 280 nm, with a flow rate of 1 mL/min and a column temperature of 40 °C. The chromatogram was recorded, the total mass of rapamycin in the liquid in the simulated blood vessel, simulated blood vessel, the liquid in the *in vitro* simulated transportation equipment and the balloon after dilation were calculated, and the percentage of rapamycin in each part with respect to the total mass was calculated.

The results were shown in Table 3.

**Table 3 Experimental results of in vitro simulated transport**

| Group | Residual drug of the balloon /% | Loss during transportation /% | Transferred amount to vessel wall /% | Loss during balloon dilation and contraction /% |
|---|---|---|---|---|
| Reference Example 1 | 25.29% | 13.05% | 33.06% | 28.60% |
| Reference Example 2 | 26.28% | 10.81% | 35.45% | 27.46% |
| Reference Example 3 | 30.26% | 11.32% | 31.74% | 26.68% |
| Example 4 | 20.26% | 15.65% | 40.77% | 23.32% |
| Example 5 | 21.27% | 14.46% | 44.16% | 20.11% |
| Example 6 | 23.29% | 14.05% | 43.06% | 19.60% |
| Example 7 | 18.81% | 19.63% | 30.15% | 31.41% |
| Example 8 | 25.84% | 20.61% | 31.72% | 21.83% |
| Example 9 | 18.69% | 20.12% | 30.44% | 30.75% |
| Example 10 | 15.84% | 21.58% | 30.19% | 32.39% |
| Comparative Example 1 | 9.47% | 53.14% | 7.22% | 30.17% |

The results in the above table showed that, compared with Comparative Example 1, the amount of drug transferred to the blood vessel wall by the drug coating of Examples 1-10 of the present invention was significantly increased, and the amount of loss during transportation was significantly reduced.

The comparison between Reference Example 1 and Example 7 showed that, in the preferred solution of the present invention, the amount of the drug transferred to the blood vessel wall can be further increased by adding a binder to the core-shell structure layer and adding a binder to the nanoparticle layer, and losses during the delivery process and losses during balloon dilation -contraction were reduced.

The comparison between Reference Example 1 and Examples 9-10 showed that, in the preferred solution of the present invention, the core-shell structure layer, the drug nanoparticle layer and the positively-charged hydrophobic modified layer are sequentially attached to the surface of the substrate, as a result the amount of drug transferred to the blood vessel wall can be further increased, and the loss during delivery and the loss during balloon dilation -contraction were reduced.

The comparison of Examples 1-6 showed that in the preferred solution of the present invention, by using phytanic acid and dioleoyl phosphatidylethanolamine as the hydrophobic substance and the positively-charged modified substance, the amount of the drug transferred to the blood vessel wall can be further increased, and the loss during delivery and the loss during balloon dilation -contraction were reduced.

The comparison between Examples 1-3 and Examples 4-6 showed that, in the present invention, the amount of the drug transferred to the blood vessel wall can be further increased by controlling the mass ratio of the positively-charged modified substance to the hydrophobic substance within a preferred range.

### EXPERIMENTAL EXAMPLE 3 In vivo Pharmacokinetics Experiment

Twelve white pigs were randomly divided into 2 groups, an experimental group and a control group, with 6 white pigs in each group. The experimental group white pigs received 1 interventional drug balloon of Example 5 in each of coronary vessels LAD (left anterior descending branch), LCX (left circumflex branch) and RCA (right coronary artery) respectively, and the control group white pigs received 1 interventional drug balloon of Comparative Example 1 in each of coronary vessels LAD (left anterior descending branch), LCX (left circumflex branch) and RCA (right coronary artery). Each balloon was dilated once for 60S, and withdrawn. Then the animals in the experimental group and the control group were sacrificed at 0 days (post-intervention), 7 days, and 28 days, respectively. 2 animals in each group were sacrificed at each time point. Blood vessels undergoing balloon dilation were collected and stored at -80°C. The blood vessel wall tissue samples were thawed at room temperature, and the average concentration of rapamycin in the blood vessel tissue samples was tested by LC-MS/MS method. The results were shown in the following table.

**Table 4 Drug concentration/(ng/mg) in blood vessel tissue**

| | 0 day | 7 days | 28 days |
|---|---|---|---|
| Experimental Group | 215.44 | 59.61 | 6.20 |

The results of the pharmacokinetic study showed that the amount of the drug released into the vascular tissue from the drug balloon prepared in Example 5 of the present invention was significantly higher than that of the drug balloon of the comparative example.

## Claims

1. A drug-coated balloon comprising a balloon body and a drug coating attached to the outside of the balloon body, **characterized in that**, the drug coating comprises a drug active coating and a positively-charged hydrophobic modified layer which are sequentially attached to the surface of a substrate, and the positively-charged hydrophobic modified layer comprises a positively-charged modified substance and a hydrophobic substance, the drug active coating comprises a core-shell structure layer and a drug nanoparticle layer, the hydrophobic substance is phytanic acid and the positively-charged modified substance is dioleoyl phosphatidylethanolamine.

2. The drug-coated balloon of claim 1, **characterized in that**,
the core-shell structure layer comprises a core-shell structure particle, the core-shell structure particle has an inner core and an outer shell surrounding the inner core, the inner core is a drug particle, and the outer shell is a polymer shell; and
the drug nanoparticle layer comprises a drug nanoparticle.

3. The drug-coated balloon of claim 2, **characterized in that**, the core-shell structure particle has a particle size D50 of 100 nm to 9 µm, preferably 300 nm to 6 µm.

4. The drug-coated balloon of claim 2, **characterized in that**, the drug nanoparticle has a particle size D50 of 100nm to 600nm.

5. The drug-coated balloon of any one of claims 2-4, **characterized in that**, the drug coating comprises a core-shell structure layer, a drug nanoparticle layer and a positively-charged hydrophobic modified layer which are sequentially attached to the surface of the substrate.

6. The drug-coated balloon of any one of claims 2-5, **characterized in that**, the drug coating has at least one feature selected from the group consisting of A to F:
A, the core-shell structure layer comprises a binder, preferably, the binder is at least one selected from the group consisting of polyvinyl alcohol, polyvinylpyrrolidone, Tween 80, poloxamer, egg yolk lecithin, soybean lecithin and methyl cellulose, preferably, a mass ratio of the binder to the core-shell structure particle is 1:1-26;
B, the polymer shell is an amphiphilic polymer shell, and the amphiphilic polymer comprises a hydrophilic block and a hydrophobic block, wherein the hydrophilic block is polyethylene glycol or polyethylene glycol monomethyl ether, and the hydrophobic block is at least one selected from the group consisting of polyoxypropylene, polystyrene, polyamino acid, polyglycolic acid, polylactide, polycaprolactone and poly(lactic-co glycolic acid); preferably, a mass ratio of the drug particle to the polymer shell in the core-shell structure particle is 0.5-5:0.5-50;
C, the drug nanoparticle layer comprises a binder, preferably, the binder is at least one selected from the group consisting of polyvinyl alcohol, polyvinylpyrrolidone, Tween 80, poloxamer, egg yolk lecithin, soybean lecithin and methyl cellulose; preferably, a mass ratio of the binder to the drug nanoparticle is 1:1-25;
D, a ratio of the drug content in the core-shell structure layer to the drug content in the drug nanoparticle layer is 1-5:1-5; preferably 1-2:1-2;
E, the drug is a medicine for preventing and treating coronary heart disease, preferably, the medicine for preventing and treating coronary heart disease is selected from rapamycin and/or a rapamycin derivative; and
F, the drug content in the core-shell structure layer is 1-5 µg/mm²; and/or the drug content in the drug nanoparticle layer is 1-5 µg/mm².

7. The drug-coated balloon of any one of claims 1-6, **characterized in that**, the positively-charged hydrophobic modified layer has at least one feature selected from the group consisting of following (1) to (2):
(1) a mass ratio of the positively-charged substance to the hydrophobic substance is 10-20:10-20, preferably 20:10;
(2) based on the total mass of the positively-charged modified substance and the hydrophobic substance, the content of the positively-charged hydrophobic modified layer is 0.1-1 µg/mm².

8. The drug-coated balloon of any one of claims 2-6, **characterized in that**, the core-shell structure particle comprises the following raw materials: 0.5-5 parts by weight of a drug, 0.5-50 parts by weight of an amphiphilic polymer, 1-50 parts by volume of an oil phase, 100-2000 parts by volume of an aqueous solution containing an emulsifier; wherein, the proportioning relationship between parts by weight and parts by volume is g/mL; optionally, the oil phase is selected from dichloromethane, acetone and any combination thereof.

9. The drug-coated balloon of any one of claims 2-6, **characterized in that**, the drug nanoparticle comprises the following raw materials: 0.5-2 parts by weight of a drug, 5-20 parts by volume of an oil phase, and 5-100 parts by volume of an aqueous solution containing an emulsifier; wherein, the proportioning relationship between parts by weight and parts by volume is g/mL; optionally, the oil phase is selected from methanol, ethanol and any combination thereof.

10. The drug-coated balloon of claim 8 or 9, wherein the emulsifier is at least one selected from the group consisting of polyvinyl alcohol, polyvinylpyrrolidone, poloxamer, bovine serum albumin, Tween 80, poloxamer, egg yolk lecithin, soybean lecithin and methyl cellulose; and/or, the emulsifier is present in an amount of 0.01-2% (weight/volume) in the aqueous solution containing the emulsifier.

11. A method for preparing a drug-coated balloon of any one of claims 1-10, **characterized by** comprising the steps of:
step S1: spraying a drug-containing coating solution on the outside of the balloon body, and drying to form a drug active coating;
step S2: mixing a positively-charged modified substance, a hydrophobic substance and an organic solvent to prepare a coating solution of the positively-charged hydrophobic modified layer, spraying the coating solution of the positively-charged hydrophobic modified layer on the outside of the drug active coating, and drying to form the positively-charged hydrophobic modified layer, obtaining a drug-coated balloon.

12. The method for preparing a drug-coated balloon of claim 11, **characterized in that**, the organic solvent is at least one selected from the group consisting of alkanes, alcohols and water; and/or a ratio of the positively-charged modified substance by mass to the hydrophobic substance by mass to the organic solvent by volume is 10-20mg:10-20mg:5mL.

13. The method for preparing a drug-coated balloon of claim 11, **characterized in that**, the drug-containing coating solution comprises a coating solution of the core-shell structure layer and/or a coating solution of the drug nanoparticle layer;
wherein the coating solution of the core-shell structure layer is prepared by: mixing the core-shell structure particle with the organic solvent to obtain the coating solution of the core-shell structure layer; and
wherein the coating solution of the drug nanoparticle layer is prepared by: mixing the drug nanoparticle with the organic solvent to obtain the coating solution of the drug nanoparticle layer.

14. The method for preparing a drug-coated balloon of claim 13, **characterized in that**, the organic solvent is at least one selected from the group consisting of alkanes, alcohols and water; and/or, in the coating solution of the core-shell structure layer, a ratio of the added core-shell structure particle by mass to the organic solvent by volume is 2.1g:3-12mL; and/or, in the coating solution of the drug nanoparticle layer, a ratio of the added drug nanoparticle by mass to the organic solvent by volume is 150mg:3-12mL.

## Patentansprüche

1. Arzneimittelbeschichteter Ballon, umfassend einen Ballonkörper und eine an der Außenseite des Ballonkörpers angebrachte Arzneimittelbeschichtung, **dadurch gekennzeichnet, dass** die Arzneimittelbeschichtung eine arzneimittelaktive Beschichtung und eine positiv geladene hydrophobe modifizierte Schicht umfasst, die nacheinander an der Oberfläche eines Substrats angebracht sind, und die positiv geladene hydrophobe modifizierte Schicht eine positiv geladene modifizierte Substanz und eine hydrophobe Substanz umfasst, die arzneimittelaktive Beschichtung eine Kern-Schale-Strukturschicht und eine Arzneimittelnanopartikelschicht umfasst, die hydrophobe Substanz Phytansäure ist und die positiv geladene modifizierte Substanz Dioleoylphosphatidylethanolamin ist.

2. Arzneimittelbeschichteter Ballon nach Anspruch 1, **dadurch gekennzeichnet, dass**
die Kern-Schale-Strukturschicht ein Kern-Schale-Strukturpartikel umfasst, das Kern-Schale-Strukturpartikel einen inneren Kern und eine den inneren Kern umgebende äußere Schale aufweist, der innere Kern ein Arzneimittelpartikel ist und die äußere Schale eine Polymerschale ist; und
die Arzneimittelnanopartikelschicht ein Arzneimittelnanopartikel umfasst.

3. Arzneimittelbeschichteter Ballon nach Anspruch 2, **dadurch gekennzeichnet, dass** das Kern-Schale-Strukturpartikel eine Partikelgröße D50 von 100 nm bis 9 µm, vorzugsweise 300 nm bis 6 µm, aufweist.

4. Arzneimittelbeschichteter Ballon nach Anspruch 2, **dadurch gekennzeichnet, dass** das Arzneimittelnanopartikel eine Partikelgröße D50 von 100 nm bis 600 nm aufweist.

5. Arzneimittelbeschichteter Ballon nach einem der Ansprüche 2-4, **dadurch gekennzeichnet, dass** die Arzneimittelbeschichtung eine Kern-Schale-Strukturschicht, eine Arzneimittelnanopartikelschicht und eine positiv geladene hydrophobe modifizierte Schicht umfasst, die nacheinander an der Oberfläche des Substrats angebracht sind.

6. Arzneimittelbeschichteter Ballon nach einem der Ansprüche 2-5, **dadurch gekennzeichnet, dass** die Arzneimittelbeschichtung wenigstens ein Merkmal aufweist, das aus der Gruppe ausgewählt ist, die aus A bis F besteht:
A, die Kern-Schale-Strukturschicht umfasst ein Bindemittel, vorzugsweise ist das Bindemittel wenigstens eines, das aus der Gruppe ausgewählt ist, bestehend aus Polyvinylalkohol, Polyvinylpyrrolidon, Tween 80, Poloxamer, Eigelblecithin, Sojalecithin und Methylcellulose, wobei vorzugsweise ein Massenverhältnis des Bindemittels zu dem Kern-Schale-Strukturpartikel 1 : 1-26 beträgt;
B, die Polymerschale ist eine amphiphile Polymerschale und das amphiphile Polymer umfasst einen hydrophilen Block und einen hydrophoben Block, wobei der hydrophile Block Polyethylenglykol oder Polyethylenglykolmonomethylether ist und der hydrophobe Block wenigstens einer ist, der aus der Gruppe ausgewählt ist, bestehend aus Polyoxypropylen, Polystyrol, Polyaminosäure, Polyglykolsäure, Polylactid, Polycaprolacton und Poly(Milchsäure-co-Glykolsäure); vorzugsweise beträgt ein Massenverhältnis des Arzneimittelpartikels zu der Polymerschale in dem Kern-Schale-Strukturpartikel 0,5-5 : 0,5-50;
C, die Arzneimittelnanopartikelschicht umfasst ein Bindemittel, vorzugsweise umfasst das Bindemittel wenigstens eines, das aus der Gruppe ausgewählt ist, bestehend aus Polyvinylalkohol, Polyvinylpyrrolidon, Tween 80, Poloxamer, Eigelblecithin, Sojalecithin und Methylcellulose; vorzugsweise beträgt ein Massenverhältnis des Bindemittels zu dem Arzneimittelnanopartikel 1 : 1-25;
D, das Verhältnis des Arzneimittelgehalts in der Kern-Schale-Strukturschicht zu dem Arzneimittelgehalt in der Arzneimittelnanopartikelschicht beträgt 1-5 : 1-5, vorzugsweise 1-2 : 1-2;
E, das Arzneimittel ist ein Medikament für die Vorbeugung und Behandlung von koronaren Herzerkrankungen, vorzugsweise ist das Medikament für die Vorbeugung und Behandlung von koronaren Herzerkrankungen aus Rapamycin und/oder einem Rapamycinderivat ausgewählt; und
F, der Arzneimittelgehalt in der Kern-Schale-Strukturschicht beträgt 1-5 µg/mm²; und/oder der Arzneimittelgehalt in der Arzneimittelnanopartikelschicht beträgt 1-5 µg/mm².

7. Arzneimittelbeschichteter Ballon nach einem der Ansprüche 1-6, **dadurch gekennzeichnet, dass** die positiv geladene hydrophobe modifizierte Schicht wenigstens ein Merkmal aufweist, das aus der Gruppe ausgewählt ist, bestehend aus den folgenden (1) bis (2):
(1) ein Massenverhältnis der positiv geladenen Substanz zu der hydrophoben Substanz beträgt von 10-20 : 10-20, vorzugsweise 20 : 10;
(2) auf Grundlage der Gesamtmasse der positiv geladenen modifizierten Substanz und der hydrophoben Substanz beträgt der Gehalt der positiv geladenen hydrophoben modifizierten Schicht 0,1-1 µg/mm².

8. Arzneimittelbeschichteter Ballon nach einem der Ansprüche 2-6, **dadurch gekennzeichnet, dass** das Kern-Schale-Partikel die folgenden Rohstoffe umfasst: 0,5-5 Gewichtsteile eines Arzneimittels, 0,5-50 Gewichtsteile eines amphiphilen Polymers, 1-50 Volumenteile einer Ölphase, 100-2000 Volumenteile einer wässrigen Lösung, die einen Emulgator enthält; wobei die Aufteilungsbeziehung zwischen Gewichtsteilen und Volumenteilen g/ml beträgt; optional die Ölphase aus Dichlormethan, Aceton und einer beliebigen Kombination davon ausgewählt ist.

9. Arzneimittelbeschichteter Ballon nach einem der Ansprüche 2-6, **dadurch gekennzeichnet, dass** das Arzneimittelnanopartikel die folgenden Rohstoffe umfasst: 0,5-2 Gewichtsteile eines Arzneimittels, 5-20 Volumenteile einer Ölphase und 5-100 Volumenteile einer wässrigen Lösung, die einen Emulgator enthält; wobei die Aufteilungsbeziehung zwischen Gewichtsteilen und Volumenteilen g/ml beträgt; optional die Ölphase aus Methanol, Ethanol und einer beliebigen Kombination davon ausgewählt ist.

10. Arzneimittelbeschichteter Ballon nach Anspruch 8 oder 9, wobei der Emulgator wenigstens eines ist, das aus der Gruppe ausgewählt ist, die aus Polyvinylalkohol, Polyvinylpyrrolidon, Poloxamer, Rinderserumalbumin, Tween 80, Poloxamer, Eigelblecithin, Sojalecithin und Methylcellulose besteht; und/oder der Emulgator in einer Menge von 0,01-2 % (Gewicht/Volumen) in der wässrigen Lösung vorhanden ist, die den Emulgator enthält.

11. Verfahren zum Herstellen eines arzneimittelbeschichteten Ballons nach einem der Ansprüche 1-10, **gekennzeichnet durch** das Umfassen der Schritte:
Schritt S1: Aufsprühen einer arzneimittelhaltigen Beschichtungslösung auf die Außenseite des Ballonkörpers und Trocknen zum Ausbilden einer arzneimittelaktiven Beschichtung;
Schritt S2: Mischen einer positiv geladenen modifizierten Substanz, einer hydrophoben Substanz und eines organischen Lösungsmittels, um eine Beschichtungslösung der positiv geladenen hydrophoben modifizierten Schicht herzustellen, Aufsprühen der Beschichtungslösung der positiv geladenen hydrophoben modifizierten Schicht auf die Außenseite der arzneimittelaktiven Beschichtung und Trocknen zum Ausbilden der positiv geladenen hydrophoben modifizierten Schicht, wodurch ein arzneimittelbeschichteter Ballon erhalten wird.

12. Verfahren zum Herstellen eines arzneimittelbeschichteten Ballons nach Anspruch 11, **dadurch gekennzeichnet, dass** das organische Lösungsmittel wenigstens eines ist, das aus der Gruppe ausgewählt ist, bestehend aus Alkanen, Alkoholen und Wasser; und/oder ein Verhältnis der positiv geladenen modifizierten Substanz nach Masse zu der hydrophoben Substanz nach Masse zu dem organischen Lösungsmittel nach Volumen 10-20 mg : 10-20 mg : 5 ml beträgt.

13. Verfahren zum Herstellen eines arzneimittelbeschichteten Ballons nach Anspruch 11, **dadurch gekennzeichnet, dass** die arzneimittelhaltige Beschichtungslösung eine Beschichtungslösung der Kern-Schale-Strukturschicht und/oder eine Beschichtungslösung der Arzneimittelnanopartikelschicht umfasst;
wobei die Beschichtungslösung der Kern-Schale-Strukturschicht hergestellt wird durch:
Mischen des Kern-Schale-Strukturpartikels mit dem organischen Lösungsmittel, um die Beschichtungslösung der Kern-Schale-Strukturschicht zu erhalten; und
wobei die Beschichtungslösung der Arzneimittelnanopartikelstrukturschicht hergestellt wird durch: Mischen des Arzneimittelnanopartikels mit dem organischen Lösungsmittel, um die Beschichtungslösung der Arzneimittelnanopartikelstrukturschicht zu erhalten.

14. Verfahren zum Herstellen eines arzneimittelbeschichteten Ballons nach Anspruch 13, **dadurch gekennzeichnet, dass** das organische Lösungsmittel wenigstens eines ist, das aus der Gruppe ausgewählt ist, bestehend aus Alkanen, Alkoholen und Wasser; und/oder in der Beschichtungslösung der Kern-Schale-Strukturschicht, ein Verhältnis des hinzugefügten Kern-Schale-Strukturpartikels nach Masse zu dem organischen Lösungsmittel nach Volumen 2,1 g : 3-12 ml beträgt; und/oder in der Beschichtungslösung der Arzneimittelnanopartikelschicht ein Verhältnis des hinzugefügten Arzneimittelnanopartikels nach Masse zu dem organischen Lösungsmittel nach Volumen 150 mg : 3-12 ml beträgt.

## Revendications

1. Ballonnet enrobé de médicament comprenant un corps de ballonnet et un enrobage de médicament fixé sur l'extérieur du corps de ballonnet, **caractérisé en ce que** l'enrobage de médicament comprend un enrobage actif de médicament et une couche modifiée hydrophobe chargée positivement qui sont fixés successivement sur la surface d'un substrat, et la couche modifiée hydrophobe chargée positivement comprend une substance modifiée chargée positivement et une substance hydrophobe, l'enrobage actif de médicament comprend une couche de structure noyau-enveloppe et une couche de nanoparticule de médicament, la substance hydrophobe est l'acide phytanique et la substance modifiée chargée positivement est la dioléoyl phosphatidyléthanolamine.

2. Ballonnet enrobé de médicament selon la revendication 1, **caractérisé en ce que**
la structure noyau-enveloppe comprend une particule de structure noyau-enveloppe, la particule de structure noyau-enveloppe a un noyau interne et une enveloppe externe entourant le noyau interne, le noyau interne est une particule de médicament, et l'enveloppe externe une enveloppe en polymère ; et
la couche de nanoparticule de médicament comprend une nanoparticule de médicament.

3. Ballonnet enrobé de médicament selon la revendication 2, **caractérisé en ce que** la particule de structure noyau-enveloppe a une taille de particule D50 de 100 nm à 9 µm, de préférence de 300 nm à 6 µm.

4. Ballonnet enrobé de médicament selon la revendication 2, **caractérisé en ce que** la nanoparticule de médicament a une taille de particule D50 de 100 nm à 600 nm.

5. Ballonnet enrobé de médicament selon l'une quelconque des revendications 2 à 4, **caractérisé en ce que** l'enrobage de médicament comprend une couche de structure noyau-enveloppe, une couche de nanoparticule de médicament et une couche modifiée hydrophobe chargée positivement qui sont fixées successivement sur la surface du substrat.

6. Ballonnet enrobé de médicament selon l'une quelconque des revendications 2 à 5, **caractérisé en ce que** l'enrobage de médicament a au moins une caractéristique choisie dans le groupe constitué par A à F :
A, la couche de structure noyau-enveloppe comprend un liant, de préférence, le liant est au moins un élément choisi dans le groupe constitué par le polyalcool vinylique, la polyvinylpyrrolidone, le Tween 80, un poloxamère, la lécithine de jaune d'œuf, la lécithine de soja et la méthyl cellulose, de préférence, un rapport en masse entre le liant et la particule de structure noyau-enveloppe est de 1:1 à 26 ;
B, l'enveloppe en polymère est une enveloppe en polymère amphiphile, et le polymère amphiphile comprend un bloc hydrophile et un bloc hydrophobe, dans lequel le bloc hydrophile est du polyéthylène glycol et en monométhyl éther de polyéthylène glycol, et le bloc hydrophobe est au moins un élément choisi dans le groupe constitué par le polyoxypropylène, le polystyrène, le polyacide aminé, le polyacide glycolique, le polylactide, la polycaprolactone et le poly(acide lactique-co-glycolique) ; de préférence, un rapport en masse entre la particule de médicament et l'enveloppe en polymère dans la particule de structure noyau-enveloppe est de 0,5 à 5:0,5 à 50 ;
C, la couche de nanoparticule comprend un liant, de préférence, le liant est au moins un élément choisi dans le groupe constitué par le polyalcool vinylique, la polyvinylpyrrolidone, le Tween 80, un poloxamère, la lécithine de jaune d'œuf, la lécithine de soja et la méthyl cellulose ; de préférence, un rapport en masse entre le liant et la nanoparticule de médicament est de 1:1 à 25 ;
D, un rapport entre la teneur en médicament dans la couche de structure noyau-enveloppe et la teneur en médicament dans la couche de nanoparticule de médicament est de 1 à 5:1 à 5 ; de préférence de 1 à 2:1 à 2 ;
E, le médicament est une substance médicamenteuse destinée à prévenir et traiter une coronaropathie, de préférence, la substance médicamenteuse destinée à prévenir et traiter une coronaropathie est sélectionnée parmi la rapamycine et/ou un dérivé de rapamycine ; et
F, la teneur en médicament dans la couche de structure noyau-enveloppe est de 1 à 5 µg/mm² ; et/ou la teneur en médicament dans la couche de nanoparticule de médicament est de 1 à 5 µg/mm².

7. Ballonnet enrobé de médicament selon l'une quelconque des revendications 1 à 6, **caractérisé en ce que** la couche modifiée hydrophobe chargée positivement a au moins une caractéristique choisie dans le groupe constitué par (1) et (2) :
(1) un rapport en masse entre la substance chargée positivement et la substance hydrophobe est de 10 à 20: 10 à 20, de préférence de 20: 10 ;
(2) sur la base de la masse totale de la substance modifiée chargée positivement et de la substance hydrophobe, la teneur en couche modifiée hydrophobe chargée positivement est de 0,1 à 1 µg/mm².

8. Ballonnet enrobé de médicament selon l'une quelconque des revendications 2 à 6, **caractérisé en ce que** la particule de structure noyau-enveloppe comprend les matières premières suivantes : 0,5 à 5 parties en poids d'un médicament, 0,5 à 50 parties en poids d'un polymère amphiphile, 1 à 50 parties en volume d'une phase huileuse, 100 à 2000 parties en volume d'une solution aqueuse contenant un émulsifiant ; dans lequel la relation de dosage entre les parties en poids et les parties en volume est g/mL ; éventuellement, la phase huileuse est choisie parmi le dichlorométhane, l'acétone et toute combinaison de ceux-ci.

9. Ballonnet enrobé de médicament selon l'une quelconque des revendications 2 à 6, **caractérisé en ce que** la nanoparticule de médicament comprend les matières premières suivantes : 0,5 à 2 parties en poids d'un médicament, 5 à 20 parties en volume d'une phase huileuse, et 5 à 100 parties en volume d'une solution aqueuse contenant un émulsifiant ; dans lequel la relation de dosage entre les parties en poids et les parties en volume est g/mL ; éventuellement, la phase huileuse est choisie parmi le méthanol, l'éthanol et toute combinaison de ceux-ci.

10. Ballonnet enrobé de médicament selon la revendication 8 ou 9, dans lequel l'émulsifiant est au moins un élément choisi dans le groupe constitué par le polyalcool vinylique, la polyvinylpyrrolidone, la sérum albumine bovine, le Tween 80, un poloxamère, la lécithine de jaune d'œuf, la lécithine de soja et la méthyl cellulose ; et/ou l'émulsifiant est présent dans une quantité de 0,01 à 2 % (poids/volume) dans la solution aqueuse contenant l'émulsifiant.

11. Procédé de préparation d'un ballonnet enrobé de médicament de l'une quelconque des revendications 1 à 10, **caractérisé en ce qu'**il comprend les étapes consistant à :
étape S1 : pulvériser une solution d'enrobage contenant un médicament sur l'extérieur du corps de ballonnet, et sécher pour former un enrobage actif de médicament ;
étape S2 : mélanger une substance modifiée chargée positivement, une substance hydrophobe et un solvant organique pour préparer une solution d'enrobage de la couche modifiée hydrophobe chargée positivement, pulvériser la solution d'enrobage de la couche modifiée hydrophobe chargée positivement sur l'extérieur de l'enrobage actif de médicament, et sécher pour former la couche modifiée hydrophobe chargée positivement, et ainsi obtenir un ballonnet enrobé de médicament.

12. Procédé de préparation d'un ballonnet enrobé de médicament selon la revendication 11, **caractérisé en ce que** le solvant organique est au moins un élement choisi dans le groupe constitué par les alcanes, les alcools et l'eau ; et/ou un rapport entre la substance modifiée chargée positivement en masse, la substance hydrophobe en masse et le solvant organique en volume est de 10 à 20 mg:10 à 20 mg:5 mL.

13. Procédé de préparation d'un ballonnet enrobé de médicament selon la revendication 11, **caractérisé en ce que** la solution d'enrobage contenant un médicament comprend une solution d'enrobage de la couche de structure noyau-enveloppe et/ou une solution d'enrobage de la couche de nanoparticule de médicament ;
dans lequel la solution d'enrobage de la couche de structure noyau-enveloppe est préparée par : mélange de la particule de structure noyau-enveloppe avec le solvant organique pour obtenir la solution d'enrobage de la couche de structure noyau-enveloppe ; et
dans lequel la solution d'enrobage de la couche de nanoparticule de médicament est préparée par : mélange de la nanoparticule de médicament avec le solvant organique pour obtenir la solution d'enrobage de la couche de nanoparticule de médicament.

14. Procédé de préparation d'un ballonnet enrobé de médicament selon la revendication 13, **caractérisé en ce que** le solvant organique est au moins un élément choisi dans le groupe constitué par les alcanes, les alcools et l'eau ; et/ou, dans la solution d'enrobage de la couche de structure noyau-enveloppe, un rapport entre la particule de structure noyau-enveloppe ajoutée en masse et le solvant organique en volume est de 2,1 g:3 à 12 mL ; et/ou, dans la solution d'enrobage de la couche de nanoparticule de médicament, un rapport entre la nanoparticule de médicament ajoutée en masse et le solvant organique en volume est de 150 mg:3 à 12 mL.
